Europäisches Patentamt

European Patent Office

Office européen des brevets

(19)

(11) Publication number: 0 289 689
A1

(12) **EUROPEAN PATENT APPLICATION**

(21) Application number: 87830170.4

(22) Date of filing: 08.05.87

(51) Int. Cl.⁴: **C07C 143/58 , C07C 143/63**

(43) Date of publication of application:
09.11.88 Bulletin 88/45

(84) Designated Contracting States:
**AT BE CH DE ES FR GB GR IT LI LU NL SE**

(71) Applicant: **APITAL PRODUZIONI INDUSTRIALI S.p.A.**
**Piazzale Principessa Clothilde, 6**
**Milano(IT)**

(72) Inventor: **Rossi, Piero**
**Via Brahms, 7**
**Bergamo(IT)**
Inventor: **Quintini, Massimo**
**Piazzale Principessa Clotilde, 6**
**Milano(IT)**

(74) Representative: **Minoja, Fabrizio**
**Studio Consulenza Brevettuale Via Rossini, 8**
**I-20122 Milano(IT)**

(54) Process for the preparation of amino aryl sulfonic acids.

(57) A process for the preparation of aminoarylsul-
fonic acids is described by heating of sulfolane
solutions of arylamine's acid sulfates. Particularly,
sulfanilic acid is obtained from aniline in high yields
and purity.

EP 0 289 689 A1

# PROCESS FOR THE PREPARATION OF AMINOARYLSULFONIC ACIDS

The present invention refers to a process for preparing aminoarylsulfonic acids.

The aminoarylsulfonic acids are widely used for the preparation of dyes and other chemical products. The oldest and most common process for such a kind of products is the high temperature heating of the acid sulfate of the aromatic amine to be sulfonated.

In order to attain the high temperatures required, ovens with outer heating or provided with hot gases circulation are usually used.

The process in ovens asks for the use of trays to be filled and emptied one by one with great manpower waste and serious problems of environmental hygiene.

An improvement is provided by the "tunnel oven" process, as disclosed in FIAT, final report 1313 or in German Dyestuff and Dyes Intermediates, vol. 1 255/56, 1948, yielding anyhow bad quality products, particularly as far as colour and charcoal products formation is concerned, because of the oxidizing action of air or of the over-heating on the wall.

A further improvement is claimed in GB 1435100 using an heating system based on hot gases flow in order to improve the thermal exchange and GB 1500343 claiming a continuous "oven" process and with air exclusion.

All the products obtained according to the above processes are anyway dark in colour, sometimes almost black, and they ask for a further purification process in order to obtain the purity degree asked for by most uses.

The heating of the amine sulfates in an inert, high-boiling, non-basic solvent, substantially petroleum fractions, is disclosed in the German patent No. 549136. The so obtained product is however accompanied by charcoal products and asks for a subsequent purification step, generally comprising the extraction of the sulfonate with alkali and its subsequent re-precipitation with acids.

It has now been found that, carrying out the reaction in sulfolane, it is possible to obtain, at temperatures lower than its boiling point, and with short enough reaction times, particularly pure sulfonated arylamines, particularly sulfaminic acid, also according to the used isolation method, so as not to ask for further purification steps.

In comparison with the known methods, and particularly with the method disclosed in DE 549136, higher yields and completely white final products, requiring no other treatments, are obtained.

On the contrary, the products obtained according to DE 549136 (Comparative Example) show a remarkable darkening, so as to prevent or to limit the use in the intended applications.

The technical result obtained by the use of a nonconventional solvent such as sulfolane should be therefore considered surprising.

The sulfolane, differently from the conventional solvents such as those used in DE 549136, is able to dissolve, at warm temperature, the amine sulfate, so that the reaction occurs in acid solution rather than in suspension.

As aromatic amines, amines whose acid sulfates is soluble in sulfolane at the reaction temperatures, whereas the obtained sulfonic acid is insoluble, are preferably, but not exclusively, used.

As a general procedure it is better to add first the amine to sulfolane, then the sulphuric acid in stoichiometric amount with respect to the amine.

The neutralization heat may be used to bring the mixture to the reaction temperature.

The temperature suited for a fast reaction rate is 160 to 210°C, preferably 180-200°C.

During the reaction, water is formed which is removed by distillation from the reactor.

When the reaction is over, which takes from 1 to 4 hours, it is either possible to evaporate directly the sulfolane in a rotary evaporator under vacuum or with similar apparatus, or, whenever a product of the best quality is desired, to filtrate first sulfolane and drying therefore the cake itself with a suitable dryer.

The operative conditions of the dryer in this case may be temperatures not higher than 180°C with a residual vacuum of 10/12 mmHg, or less drastic conditions whenever it is preferred to remove first the sulfolane from the cake by means of water or other suitable solvent with lower boiling point.

## EXAMPLE 1

697.5 G of aniline are added to 2.250 g of sulfolane. 750 G of 98% sulfuric acid are added dropwise in one hour. The mixture is heated to 180-190°C and stirred for 1 hour at 180-190°C. It is cooled to 30-40°C. The mixture is filtered, washed on the filter with 500 g of sulfolane. The cake is dried under vacuum of 30-40 torr at 160-170°C.

1.233 G of white sulfanilic acid are obtained having an assay higher than 99% (95% yield).

## EXAMPLE 2

558 G of aniline are added to 1.800 g of sulfolane. 600 G of 98% sulfuric acid are added dropwise in 1 hour. The mixture is heated to 180-190°C and stirred for 1 hour at 180-190°C. All the sulfolane is then distilled under vacuum of 30-40 torr at 160-170°C.

1.034.6 G of sulfanilic acid, having almost grey colour with 98.3% assay (98% yield) are obtained as distillation residue.

## EXAMPLE 3

127.5 G of p-chloroaniline are added to 300 g of sulfolane. 100 G of 98% sulfuric acid are added dropwise in 1 hour. The mixture is heated to 200-205°C and stirred at 200-205° for 4 hours. The mixture is cooled to 30-40°C, filtered, washed with sulfolane and then with water.

136 G of chloro aminobenzenesulfonic acid, with 95% assay, are obtained after drying.

Yield: 62.3%.

## EXAMPLE 4

107 G of o-toluidine are added to 300 g of sulfolane. 100 G of 98% sulfuric acid are added dropwise. The mixture is heated to 190-200°C, stirred for 2 hours at 190-200°C, cooled to 30-40°C, filtered, washed with sulfolane and then with water.

156 G of aminotoluenesulfonic acid are obtained.

Yield: 83.4%

## COMPARATIVE EXAMPLE

279 G of aniline are dissolved in 900 ml of petroleum fraction with boiling point higher than 200°C.

300 G of sulfuric acid are added and the mixture is heated for 5 hours at 190-195°C. 450 G of 30% sodium hydroxide and 1.500 ml of water are added to the resulting mass, markedly coloured and with charcoal products in suspension.

In order to allow the separation of the phases, the mixture is filtered from carbon deposits and the acqueous phase is allowed to separate. After separation, the acqueous phase is acidified with 352 g of 35% hydrochloric acid and the sulfanilic acid which precipitates is collected by filtration, washed with water and dried.

480 G of dark product, having assay (by diazotation) of 93.7%, are obtained.

The yield is 86.6% of the theory.

## Claims

1. Process for the preparation of arylaminosulfonic acids by heating arylamines acid sulfates characterized in that sulfolane is used as solvent.

2. Process according to claim 1, wherein the reaction temperature ranges from 160 to 210°C.

3. Process according to claim 1 or 2, wherein the sulfolane is removed by evaporation under reduced pressure at the end of the reaction, preferably at a residual pressure lower than 50 torr and at a temperature equal or higher than the sulfolane boiling temperature, at the pre-established residual pressure.

4. Process according to any one of the previous claims, wherein the arylaminosulfonic acid is isolated from the reaction mixture by filtration and subsequently deprived of sulfolane by drying.

5. Process according to claim 4, wherein the cake obtained from the sulfolane filtration is subsequently washed with water or other suitable solvent and finally dried.

6. Process according to any one of the previous claims, wherein the arylamine is aniline and the arylaminosulfonic acid is sulfanilic acid.

7. Arylaminosulfonic acids obtainable by the processes of claims 1-6.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 063 269  (BAYER AG) <br> * Examples; claims * <br> ----- | 1-7 | C 07 C 143/58 <br> C 07 C 143/63 |
| | | | TECHNICAL FIELDS SEARCHED (Int. Cl.4) |
| | | | C 07 C 143/00 |

The present search report has been drawn up for all claims

| Place of search | Date of completion of the search | Examiner |
|---|---|---|
| THE HAGUE | 18-08-1988 | PAUWELS G.R.A. |

EPO FORM 1503 03.82 (P0401)